# EUROPÄISCHE PATENTSCHRIFT

(11) **EP 2 125 771 B1**
(45) Veröffentlichungstag und Bekanntmachung des Hinweises auf die Patenterteilung: **20.03.2013**
(21) Anmeldenummer: 07803047.5
(22) Anmeldetag: 30.08.2007
(51) Int. Cl.: C07D 319/12

(54) **VERFAHREN ZUR HERSTELLUNG VON TETRAMETHYLGLYCOLID**
PROCESS FOR PREPARING TETRAMETHYL GLYCOLIDE
PROCÉDÉ DE FABRICATION DE TÉTRAMÉTHYLGLYCOLIDE

(30) Priorität: 22.11.2006 DE 102006055427
(43) Veröffentlichungstag der Anmeldung: 02.12.2009
(73) Patentinhaber: Evonik Röhm GmbH, 64293 Darmstadt (DE)
(72) Erfinder: VOGEL, Bernd, 65193 Wiesbaden (DE); MAY, Alexander, 64289 Darmstadt (DE); SIEGERT, Hermann, 64342 Seeheim-Jugenheim (DE)
(86) Internationale Anmeldenummer: PCT/EP2007/059038
(87) Internationale Veröffentlichungsnummer: WO 2008/061821

(56) Entgegenhaltungen:
- EP-A- 0 487 853
- WO-A-95/09142
- WO-A-2005/077878
- GOLOMB A. ET AL.: "Studies in pyrolysis. Part XVII. The acyl derivatives and lactides of some -hydroxy-acids" J. CHEM. SOC., 1962, Seiten 838-847, XP009092793

## Beschreibung

Die vorliegende Erfindung betrifft Verfahren zur Herstellung von Tetramethylglycolid umfassend die Umsetzung von 2-Hydroxyisobuttersäure.

Cyclische Ester werden seit längerem insbesondere zur Herstellung von biologisch abbaubaren Polymeren eingesetzt, wobei die Herstellung dieser Ester aus α-Hydroxycarbonsäuren seit längerem Stand der Technik ist. Beispielsweise beschreibt die Druckschrift DE 26 78 26 ein Verfahren zur Herstellung eines Lactids, bei dem Milchsäure langsam auf eine Temperatur von 200°C erhitzt und das dabei entstehende Lactid, vorteilhaft im Vakuum abdestilliert wird. Bei diesem Verfahren entstehen insbesondere Oligomere und Polymere der Milchsäure. Dementsprechend ist das Verfahren mit vielen Nachteilen behaftet. Hierzu gehören insbesondere die geringe Reinheit der erhaltenen Produkte sowie die geringe Ausbeute.

Des Weiteren sind Verfahren zur Herstellung von cyclischen Estern bekannt, bei denen ein Polyester, insbesondere Polylactide unter Bildung von cyclischen Estern depolymerisiert werden. Derartige Verfahren sind beispielsweise Gegenstand der Druckschrift DE-A-37 08 915, wobei im einleitenden Teil dieser Schrift weiterer Stand der Technik zitiert wird. Nachteilig an diesen Verfahren ist insbesondere, dass zunächst Polymere hergestellt und aufgereinigt werden müssen. Daher sind diese Verfahren aufgrund der eingesetzten Ausgangsstoffe relativ teuer.

Darüber hinaus beschreibt das Dokument WO 95/09142 Verfahren zur Herstellung von cyclischen Estern, bei denen zunächst eine wässrige Phase einer α-Hydroxycarbonsäure hergestellt wird. Die α-Hydroxycarbonsäure wird durch eine Extraktion mit einem organischen Lösungsmittel der wässrigen Phase entzogen. Die organische Phase wird anschließend mit einem weiteren Lösungsmittel, das einen höheren Siedepunkt als das Extraktionsmittel aufweist, versetzt. Das Extraktionsmittel wird in einem weiteren Schritt entfernt. Anschließend wird die in dieser Reaktionsmischung enthaltene α-Hydroxycarbonsäure zum cyclischen Ester umgesetzt, wobei der Anteil an Oligomeren und Polymere geringer als 20 Gew.-% gehalten wird. Danach kann der erhaltene cyclische Ester von Nebenprodukten durch Extraktion oder Kristallisation abgetrennt werden. Dieses Verfahren ist sehr aufwendig, da viele Schritte notwendig sind, um die erhaltenen Produkte aufzureinigen. Darüber hinaus muss der Gehalt an Oligomeren und/oder Polymeren niedrig gehalten werden. Dies beinhaltet jedoch, dass die Konzentration an Edukt relativ gering gehalten werden muss. Daher sind sehr hohe Mengen an Lösungsmitteln abzutrennen.

Das Dokument J. Chem. Soc., 1962 , 838-847, offenbart ein Verfahren zur Herstellung von Tetramethylglycolid aus 2-Hydroxyisobuttersäure in einer Destille, ohne Lösungsmittel bei einer Temperatur von 190-220°C.

Weiterhin beschreibt das Dokument EP-A-0 834 511 die Herstellung und Aufreinigung von cyclischen Estern. Die cyclischen Ester werden hierbei entweder durch Depolymerisation von Polymeren oder durch Cyclisierung von α-Hydroxycarbonsäuren in Lösung erhalten. Der entscheidende Schritt ist die Umsetzung des erhaltenen Reaktionsprodukts mit Orthoestern, um den Gehalt an Wasser und Säure in dem Produkt zu verringern. Dieses Verfahren führt zu einem guten Produkt. Allerdings sind die einzusetzenden Orthoester relativ teuer, so dass das Gesamtverfahren verbessert werden kann.

Obwohl die in den zuvor dargelegten Dokumenten beschriebenen Verfahren bereits zur Herstellung von cyclischen Estern eingesetzt werden können, besteht ein permanentes Bedürfnis diese Verfahren weiter zu verbessern, um die Herstellungskosten zu senken und die Ausbeuten zu verbessern.

In Anbetracht des Standes der Technik ist es nun Aufgabe der vorliegenden Erfindung Verfahren zur Herstellung von cyclischen Estern zur Verfügung zu stellen, die besonders einfach, kostengünstig und mit hoher Ausbeute durchgeführt werden können. Ein besonderes Problem bestand insbesondere darin ein Verfahren zu schaffen, das flexibel zur Herstellung von unterschiedlichen Produkten eingesetzt werden kann, um so eine hohe Auslastung der verwendeten Produktionsanlagen zu gewährleisten.

Gelöst werden diese sowie weiter nicht explizit genannte Aufgaben, die jedoch aus den hierin einleitend diskutierten Zusammenhängen ohne weiteres ableitbar oder erschließbar sind, durch ein Verfahren mit allen Merkmalen des Patentanspruchs 1. Zweckmäßige Abwandlungen der erfindungsgemäßen Verfahren werden in Unteransprüchen unter Schutz gestellt.

Gegenstand der vorliegenden Erfindung ist dementsprechend ein Verfahren zur Herstellung von Tetramethylglycolid, dadurch gekennzeichnet, dass eine Zusammensetzung, die mindestens 50 Gew.-% 2-Hydroxyisobuttersäure und/oder Tetramethylglycolid umfasst, auf eine Temperatur von mindestens 100°C erhitzt wird und die Umsetzung bei einem Druck im Bereich von 0,1 bis 0,4 bar durchgeführt wird. Das Verfahren kann besonders einfach, kostengünstig und mit hoher Ausbeute durchgeführt werden.

Zugleich, lassen sich durch die erfindungsgemäßen Verfahren eine Reihe weiterer Vorteile erzielen. Hierzu gehört unter anderem, dass das Verfahren besonders flexibel ist. Hierbei kann das Verfahren so ausgestaltet werden, dass neben Tetramethylglycolid weitere Produkte, beispielsweise Alkylmethacrylat und/oder Methacrylsäure erhalten werden können. Darüber hinaus kann das erfindungsgemäße Verfahren bei hoher Geschwindigkeit, geringem Energieeinsatz und geringen Ausbeuteverlusten durchgeführt werden.

Die Verbindung Tetramethylglycolid (3,3,6,6-Tetrametyl-1,4-dioxan-2,5-dion) ist an sich aus dem Stand der Technik bekannt und kann durch Dimerisierung von 2-Hydroxyisobuttersäure erhalten werden, wie dies beispielsweise in EP-A-0 834 511 beschrieben ist.

Erfindungsgemäß wird Tetramethylglycolid durch die Umsetzung einer Zusammensetzung erhalten, die mindestens 50 Ges.-% 2-Hydroxyisobuttersäure und/oder, Tetramethylglycolid umfasst. Vorzugsweise umfasst die Zusammensetzung mindestens 70 Gew.-% und ganz besonders bevorzugt mindestens 90 Gew.-% 2-Hydroxyisobuttersäure und/oder Tetramethylglycolid. Der Begriff,, Hydroxyisobuttersäure und/oder Tetramethylglycolid" ver deutlicht, dass die Zusammensetzung neben dem Edukt und dem Produkt nur relativ geringe Anteile an Lösungsmitteln oder anderen Substanzen umfasst. Lösungsmittel können jedoch in der Reaktionsmischung enthalten sein. Lösungsmittel mit einem niedrigen Siedepunkt können bei einer Umsetzung in einer Destille über Kopf abgetrennt werden. Diese Lösungsmittel können insbesondere dazu dienen die Zugabetemperatur der Eduktmischung niedrig zu halten. Dies kann insbesondere bei kontinuierlichen Verfahren zweckmäßig sein. Darüber hinaus kann hierdurch die Temperatur zu Beginn der Reaktion gering gehalten werden, so dass besonders geringe Anteile an unerwünschten Nebenprodukten entstehen. Lösungsmittel mit hohen Siedepunkten verbleiben bei einer Umsetzung in einer Destille vielfach im Produkt. Diese Lösungsmittel erniedrigen vielfach die Verfestigungstemperatur, so dass der Produktstrom bei relativ niedrigen Temperaturen aus der Destille abgeleitet werden kann.

Zu den geeigneten Lösungsmitteln zählen beispielsweise Alkohole, Ketone, Aldehyde, Ester, Ether, Carbonsäuren, Kohlenwasserstoffe und Mischungen dieser Lösungsmittel untereinander sowie mit weiteren Lösungsmitteln.

Zu den Kohlenwasserstofflösungsmittel gehören aliphatische, alicyclische und aromatische Kohlenwasserstoffe. Zu diesen Kohlenwasserstoffen gehören unter anderem Pentan, Hexan, insbesondere n-Hexan und 3 Methylpentan, Heptan, insbesondere n-Heptan und 3-Methylhexan, Octan, Cyclopentan, Cyclohexan, Benzol, Toluol, Xylol, Ethylbenzol.

Des Weiteren gehören zu den geeigneten Lösungsmittel Carbonsäuren und Carbonsäureester. Zu diesen zählen insbesondere Essigsäure, Ethylacetat, α-Hydroxycarbonsäuren, insbesondere 2-Hydroxyisobuttersäure und 2-Hydroxyisobuttersäuremethylester.

Zu den als Lösungsmittel verwendbaren Ketonen zählen beispielsweise Aceton, Methylethylketon, Diethylketon, Methylpropylketon, Methylisopropylketon, Methylbutylketon, Methyl-1-methylpropylketon, Methyl-2-methylpropylketon, Ethylpropylketon und andere Ketone mit jeweils 2 oder mehr Kohlenstoffatomen, bevorzugt 4 bis 12 und besonders bevorzugt 4 bis 9 Kohlenstoffatomen.

Zu den als Lösungsmittel verwendbaren Aldehyden gehören beispielsweise Acetaldehyd, Propionaldehyd, Butyraldehyd, Valeraldehyd, Benzaldehyd und andere Aldehyde mit jeweils 2 oder mehr Kohlenstoffatomen, bevorzugt 4 bis 12 und besonders bevorzugt 4 bis 9 Kohlenstoffatomen.

Zu den als Lösungsmittel verwendbaren Ether zählen unter anderem Diethylether, Di-n-propylether, Diisopropylether, Di-n-butylether, Diisobutylether und andere Ether mit jeweils 2 oder mehr Kohlenstoffatomen, bevorzugt 4 bis 12 und besonders bevorzugt 4 bis 9 Kohlenstoffatomen.

Besonders bevorzugt können Alkohole als Lösungsmittel eingesetzt werden. Zu den bevorzugten Alkoholen gehören unter anderem Alkohole mit jeweils mindestens einem Kohlenstoffatom, bevorzugt 2 bis 12 und besonders bevorzugt 4 bis 9 Kohlenstoffatomen. Die Alkohole können eine lineare, verzweigte oder cyclische Struktur aufweisen. Des Weiteren können die Alkohole aromatische Gruppen oder Substituenten, beispielsweise Halogenatome umfassen. Zu den bevorzugten Alkoholen gehören insbesondere Methanol, Ethanol, n-Propanol, iso-Propanol, n-Butanol, 1-Methyl-propanol, 2-Methyl-propanol, tert.-Butanol, n-Pentanol, 1-Methyl-butanol, 2-Methyl-butanol, 3-Methyl-butanol, 2,2-Dimethyl-propanol, n-Hexanol, 1-Methyl-pentanol, 2-Methyl-pentanol, 3-Methyl-pentanol, 4-Methyl-pentanol, 1,1-Dimethyl-butanol, 2,2-Dimethyl-butanol, 3,3-Dimethyl-butanol, 1,2-Dimethyl-butanol, n-Heptanol, 1-Methyl-hexanol, 2-Methyl-hexanol, 3-Methyl-hexanol, 4-Methyl-hexanol, 1,2-Dimethyl-pentanol, 1, 3-Dimethyl-pentanol, 1,1-Dimethyl-pentanol, 1,1, 2, 2-Tetramethyl-propanol, Benzylalkohol, n-Octanol, 2-Ethylhexanol, n-Nonanol,1-Methyl-octanol, 2-Methyl-octanol, n-Decanol, n-Undecanol, 1-Methyl-decanol, 2-Methyl-decanol, n-Dodecanol, 2,4-Diethyl-octanol, Cyclopentanol, Cyclohexanol, 4-tert.-Butyl-cyclohexanol, Cycloheptanol, Cyclododecanol, 2-(Dimethylamino)-ethanol, 3-(Dimethylamino)-propanol, 4-(Dimethylamino)-butanol, 5-(Dimethylamino)-pentanol, 6-(Dimethylamino)-hexanol, 8-(Dimethylamino)-octanol, 10-(Dimethylamino)-decanol, 12-(Dimethylamino)-dodecanol, 2-(Diethylamino)-ethanol, 3-(Diethylamino)-propanol, 4-(Diethylamino)-butanol, 5-(Diethylamino)-pentanol, 6-(Diethylamino)-hexanol, 8-(Diethylamino)-octanol, 10-(Diethylamino)-decanol, 12-(Diethylamino)-dodecanol, 2-(Di- (iso-propyl)-amino)-ethanol, 3-(Di-(iso-propyl)-amino)-propanol, 4-(Di-(iso-propyl)-amino)-butanol, 5-(Di-(iso-propyl)-amino)-pentanol, 6-Di-(iso-propyl)-amino)-hexanol, 8-(Di-(iso-propyl)-amino)-octanol, 10-(Di-(iso-propyl)-amino)-decanol, 12-(Di-(iso-propyl)-amino)-dodecanol, 2-(Dibutylamino)-ethanol, 3-(Dibutylamino)-propanol, 4-(Dibutylamino)-butanol, 5-(Dibutylamino)-pentanol, 6-(Dibutylamino)-hexanol, 8-(Dibutylamino)-octanol, 10-(Dibutylamino)-decanol, 12-(Dibutylamino)-dodecanol, 2-(Dihexylamino)-ethanol, 3-(Dihexylamino)-propanol, 4-(Dihexylamino)-butanol, 5-(Dihexylamino)-pentanol, 6-(Dihexylamino)-hexanol, 8-(Dihexylamino)-octanol, 10-(Dihexylamino)-decanol, 12-(Dihexylamino)-dodecanol, 2-(Methyl-ethyl-amino)-ethyi-, 2-(Methyl-propyl-amino)-ethanol, 2-(Methyl-iso-propylamino)- ethanol, 2-(Methyl-butyl-arnino)-ethanol, 2-(Methyl-hexyl-amino)-ethanol, 2-(Methyl-octyl-amino)-ethanol, 2-(Ethyl-propyl-amino)-ethanol, 2-(Ethyl-iso-propylamino)-ethanol, 2-(Ethyl-butyl-amino)-ethanol, 2-(Ethyl-hexyl-amino)-ethanol, 2-(Ethyl-octyl-amino)-ethanol, 3-(Methyl-ethyl-amino)-propanol, 3-(Methyl-propyl-amino)-propanol, 3-(Methyl-iso-propyl-amino)-propanol, 3-(Methyl-butyl-amino)-propanol, 3-(Methyl-hexyl-amino)-propanol, 3-(Methyl-octyl-amino)-propanol, 3-(Ethyl-propylamino)-propanol, 3-(Ethyl-iso-propyl-amino)-propanol, 3-(Ethyl-butyl-amino)-propanol, 3-(Ethyl-hexyl-amino)-propanol, 3-(Ethyl-octyl-amino)-propanol, 4-(Methyl-ethyl-amino)-butanol, 4-(Methyl-propyl-amino)-butanol, 4-(Methyl-iso-propyl-amino)-butanol, 4-(Methyl-butyl-amino)-butanol, 4-(Methyl-hexyl-amino)-butanol, 4-(Methyl-octyl-amino)-butanol, 4-(Ethyl-propyl-amino)-butanol, 4-(Ethyl-iso-propyl-amino)-butanol, 4-(Ethylbutyl-amino)-butanol, 4-(Ethyl-hexyl-amino)-butanol, 4-(Ethyl-octyl-amino)-butanol, 2-(N-Piperidinyl)-ethanol, 3-(N-Piperidinyl)-propanol, 4-(N-Piperidinyl)-butanol, 5-(N-Piperidinyl)-pentanol, 6-(N-Piperidinyl)-hexanol, 8-(N-Piperidinyl)-octanol, 10-(N-Piperidinyl)-decanol, 12-(N-Piperidinyl)-dodecanol, 2-(N-Pyrrolidinyl)-ethanol, 3-(N-Pyrrolidinyl)-propanol, 4-(N-Pyrrolidinyl)-butanol, 5-(N-,Pyrrolidinyl)-pentyl-, 6-(N-Pyrrolidinyl)-hexanol, 8-(N-Pyrrolidinyl)-octanol, 10-(N-Pyrrolidinyl)-decanol, 12-(N-Pyrrolidinyl)-dodecanol, 2-(N-Morpholino)-ethanol, 3-(N-Morpholino)-propanol, 4-(N-Morpholino)-butanol, 5-(N-Morpholino)-pentanol, 6-(N-Morpholino)-hexanol, 8-(N-Morpholino)-octanol, 10-(N-Morpholino)-decanol, 12-(N-Morpholino)-dodecanol, 2-(N'-Methyl-N-Piperazinyl)-ethanol, 3-(N'-Methyl-N-Piperazinyl)-propanol, 4-(N'-Methyl-N-Piperazinyl)-butanol, 5-(N'-Methyl-N-Piperazinyl)-pentanol, 6-(N'-Methyl-N-Piperazinyl)-hexanol, 8-(N'-Methyl-N-Piperazinyl)-octanol, 10-(N'-Methyl-N-Piperazinyl)-decanol, 12-(N'-Methyl-N-Piperazinyl)-dodecanol, 2-(N'-Ethyl-N-Piperazinyl)-ethanol, 3-(N'-Ethyl-N-Piperazinyl)-propanol, 4-(N'-Ethyl-N-Piperazinyl)-butanol, 5-(N'-Ethyl-N-Piperazinyl)-pentanol, 6-(N'-Ethyl-N-Piperazinyl)-hexanol, 8-(N'-Ethyl-N-Piperazinyl)-octanol, 10-(N'-Ethyl-N-Piperazinyl)-decanol, 12-(N'-Ethyl-N-Piperazinyl)-dodecanol, 2-(N'-iso-Propyl-N-Piperazinyl)-ethanol, 3-(N'-iso-Propyl-N-Piperazinyl)-propanol, 4-(N'-iso-Propyl-N-Piperazinyl)-butanol, 5-(N'-iso-Propyl-N-Piperazinyl)-pentanol, 6-(N'-iso-Propyl-N-Piperazinyl)-hexanol, 8-(N'-iso-Propyl-N-Piperazinyl)-octanol, 10-(N'-iso-Propyl-N-Piperazinyi)-decanol, 12-(N'-iso-Propyl-N-Piperazinyl)-dodecanol, 3-Oxa-butanol, 3-Oxa-pentanol, 2,2-Dimethyl-4-oxa-pentanol, 3,6-Dioxa-heptanol, 3,6-Dioxa-octanol, 3,6,9-Trioxa-decanol, 3,6,9-Trioxa-undecanol, 4-Oxa-pentanol, 4-Oxa-hexanol, 4-Oxa-heptanol, 4,8-Dioxa-nonanol, 4,8-Dioxa-decanol, 4,8-Dioxa-undecanol, 5-Oxa-hexanol oder 5,10-Dioxa-undecanol.

Weiterhin können ethoxylierte und/oder propoxylierte Alkohole sowie gemischtethoxylierte/propoxylierte Alkohole als Lösungsmittel eingesetzt werden, insbesondere R⁵-(O-CH₂-CH₂)ₓ-OH oder
R⁵-(O-CH(CH₃)-CH₂)ₓ-OH, beziehungsweise R⁵-(O-CH₂-CH(CH₃))x-OH,
worin
R⁵ für C₁ bis C₂₀-Alkyl und
x für eine ganze Zahl zwischen 10 und 20 steht,
oder ethoxylierte und/oder propoxylierte Aminoalkohole, wie zum Beispiel
R⁶₂N(-CH₂-CH₂-O)_{y}-H oder R⁶₂N(-CH(CH₃)-CH₂-O)_{y}-H beziehungsweise
R⁶₂N(-CH₂CH(CH₃)-O)_{y}-H,
worin y für eine ganze Zahl zwischen 1 und 4 steht. R⁶ steht für eine Alkylgruppe mit 1 - 6 Kohlenstoffatomen, wobei der Stickstoff mit den Substituenten R⁶ auch einen fünf- bis siebengliedrigen Ring bilden kann. Der Ring kann gegebenenfalls noch durch eine oder mehrere kurzkettige Alkylgruppen, wie beispielsweise Methyl, Ethyl oder Propyl, substituiert sein.

Erfindungsgemäß wird die 2-Hydroxyisobuttersäure enthaltende Zusammensetzung auf eine Temperatur von mindestens 100°C, bevorzugt mindestens 150°C und besonders bevorzugt mindestens 170°C erhitzt. Die obere Temperäturgrenze ergibt sich insbesondere aus dem Siedepunkt der Zusammensetzung, falls die Umsetzung m flüssiger Phase durchgeführt wird.

Die Umsetzung erfolgt vorzugsweise in flüssiger Phase, wobei ein Katalysator, insbesondere ein sauerer Katalysator zugegeben werden kann. Hierbei können sowohl homogene als auch heterogene Katalysatoren verwendet werden. Besonders geeignete Katalysatoren sind insbesondere anorganische Säuren, beispielsweise Schwefelsäure oder Salzsäure, sowie organische Säuren, beispielsweise Sulfonsäuren, insbesondere p-Toluolsulfonsäure sowie saure Kationenaustauscher Gemäß einem besonders bevorzugten Aspekt erfolgt die Umsetzung autokatalytisch.

Die Reaktion wird bei einem Druck im Bereich von 0,1 bis 0,4 bar durchgeführt.

Die Umsetzung kann chargenweise oder kontiuierlich durchgeführt werden, wobei kontinuierliche Verfahren bevorzugt sind. Gemäß einem besonderen Aspekt der vorliegenden Erfrirdung kann die Herstellung von Tetramethylglycolid in einer Destille erfolgen. Hierfür geeignete Destillationsanlagen sind allgemein bekannt und werden vielfach zur Trennung eingesetzt Die Verwendung einer Destille zur Durchführung der erfindungsgemäßen Umsetzung ermöglicht insbesondere, dass entstehendes Wasser aus der Reaktionsmischung durch Destillation entfernt werden kann. Diese Art das Reaktionsgleichgewicht zu beeinflussen ist besonders ökonomisch. Allerdings kann entstehendes Wasser ebenfalls durch andere Verfahren abgetrennt werden.

Die Destille kann aus jedem hierfür geeigneten Material hergestellt werden. Hierzu gehören u.a. Edelstahl sowie inerte Materialien.

Die Reaktionsdauer der erfindungsgemäßen Umsetzung hängt von der Reaktionstemperatur ab, wobei dieser Parameter in weiten Bereichen liegen kann. Bevorzugt liegt die Reaktionszeit der Umsetzung im Bereich von 5 Minuten bis 50 Stunden, besonders bevorzugt 30 Minuten bis 30 Stunden und ganz besonders bevorzugt 1 Stunde bis 10 Stunden.

Bei kontinuierlichen Verfahren beträgt die Verweilzeit vorzugsweise 5 Minuten bis 50 Stunden, besonders bevorzugt 30 Minuten bis 30 Stunden und ganz besonders bevorzugt 1 Stunde bis 10 Stunden.

Das hergestellte Tetramethylglycolid kann für einige Anwendungen unmittelbar, ohne weitere Aufreinigungsschritte eingesetzt werden. Des Weiteren kann das Tetramethylglycolid durch Destillation oder chromatographische Verfahren zusätzlich aufgereinigt werden. Hierzu können insbesondere Verfahren des Standes der Technik, wie beispielsweise in EP-A-0 834 511 beschrieben, eingesetzt werden.

Des Weiteren kann das hergestellte Tetramethylglycolid auch durch Extraktion aus der Reaktionsmischung abgetrennt werden, wobei geeignete Extrationsmittel an sich bekannt sind. Diese Mittel sollten eine geringe Mischbarkeit mit den Ausgangsmaterialien aufweisen, wobei jedoch ein hoher Anteil des Produkts in das Extraktionsmittel übergehen sollte.

Gemäß einem besonderen Aspekt kann das erfindungsgemäße Verfahren in Kombination mit einem Verfahren zur Herstellung von Alkylmethacrylaten durchgeführt werden. Daher kann das erfindungsgemäße Verfahren sehr flexibel zur Herstellung mehrerer kommerziell bedeutender Produkte dienen, wobei hierdurch eine hohe Auslastung der Anlagen erzielt werden kann.

Ein besonders bevorzugtes Verfahren kann beispielsweise folgende Schritte umfassen:
A) Bildung von Acetoncyanhydrin durch Umsetzung von Aceton mit Blausäure;
B) Hydrolyse des Acetoncyanhydrins unter Bildung von 2-Hydroxyisobuttersäureamid;
C) Alkoholyse des 2-Hydroxyisobuttersäureamids, wobei ein 2-Hydroxyisobuttersäureester erhalten wird;
D) Umesterung des 2-Hydroxyisobuttersäureesters bzw. der 2-Hydroxyisobuttersäureester mit Methacrylsäure, wobei mindestens ein Alkylmethacrylat und 2-Hydroxyisobuttersäure gebildet werden;
E) Dehydratisierung der 2-Hydroxyisobuttersäure, wobei Methacrylsäure gebildet wird.

Die erfindungsgemäße Herstellung von Tetramethylglycolid erfolgt hierbei aus einem Teil der in Schritt D) erhaltenen 2-Hydroxyisobuttersäure. Der Anteil an Tetramethylglycolid und der Anteil an Alkylmethacrylat kann beispielsweise über die Reaktionstemperatur und den Druck sowie den Wassergehalt der Umsetzung gesteuert werden. Je höher der Wassergehalt bei der Umesterung, desto größer mehr Alkohol kann bei der Umesterung freigesetzt werden, so dass ein Teil des 2-Hydroxyisobuttersäureesters durch Hydrolyse in 2-Hydroxyisobuttersäure überführt wird, aus dem nachfolgend Tetramethylglycolid gebildet werden kann. Darüber hinaus kann der Anteil an 2-Hydroxyisobuttersäure über die Temperatur gesteuert werden. Die Menge an Tetramethylglycolid, die aus der in Schritt D) hergestellten 2-Hydroxyisobuttersäure gebildet wird, kann insbesondere über die Temperatur sowie die Verweilzeit gesteuert werden.

In einem ersten Schritt wird Aceton mit Blausäure zum entsprechenden Acetoncyanhydrin umgesetzt. Diese Umsetzung erfolgt im Allgemeinen unter Verwendung einer geringen Menge an Alkali oder eines Amins als Katalysator.

In einem weiteren Schritt wird das so erhaltene Acetoncyanhydrin mit Wasser zum 2-Hydroxyisobuttersäureamid umgesetzt.

Typischerweise wird diese Umsetzung in Gegenwart eines Katalysators durchgeführt. Hierfür geeignet sind insbesondere Manganoxidkatalysatoren, wie diese beispielsweise in EP-A-0945429, EP-A-0561614 sowie EP-A-0545697 beschrieben sind. Hierbei kann das Manganoxid in Form von Mangandioxid eingesetzt werden, welches durch Behandlung von Mangansulfat mit Kaliumpermanganat unter sauren Bedingungen (vgl. Biochem.J., 50 S. 43 (1951) und J.Chem.Soc., S. 2189, 1953) oder durch elektrolytische Oxidation von Mangansulfat in wässriger Lösung erhalten wird. Im Allgemeinen wird der Katalysator vielfach in Form von Pulver oder Granulat mit einer geeigneten Korngröße eingesetzt. Des Weiteren kann der Katalysator auf einen Träger aufgebracht werden. Hierbei können insbesondere auch sogenannte Slurry-Reaktoren oder Festbett-Reaktoren eingesetzt werden, die unter anderem in EP-A-956 898 beschrieben sind.

Des Weiteren kann die Hydrolysereaktion durch Enzyme katalysiert werden. Zu den geeigneten Enzymen gehören unter anderem Nitrilhydratasen. Diese Reaktion ist beispielhaft in "Screening, Characterization and Application of Cyanide-resistant Nitrile Hydratases" Eng. Life. Sci. 2004, 4, No. 6 beschrieben.

Darüber hinaus kann die Hydrolysereaktion durch Säuren, insbesondere Schwefelsäure katalysiert werden. Dies wird unter anderem in JP Hei 4- 193845 dargelegt.

Das Wasser, welches zur Hydrolyse des Acetoncyanhydrins notwendig ist, kann vielfach als Lösungsmittel eingesetzt werden. Vorzugsweise beträgt das molare Verhältnis von Wasser zu Cyanhydrin mindestens 1, besonders bevorzugt liegt das molare Verhältnis von Wasser zu Cyanhydrin im Bereich von 0,5:1-25:1 und ganz besonders bevorzugt im Bereich von 1:1-10:1.

Das zur Hydrolyse eingesetzte Wasser kann einen hohen Reinheitsgrad aufweisen. Allerdings ist diese Eigenschaft nicht zwingend. So kann neben Frischwasser auch Brauchwasser oder Prozesswasser eingesetzt werden, welches mehr oder minder hohe Mengen an Verunreinigungen umfasst. Dementsprechend kann auch recyceltes Wasser zur Hydrolyse verwendet werden.

Des Weiteren können weitere Bestandteile in der Reaktionsmischung zur Hydrolyse des Cyanhydrins vorhanden sein. Hierzu gehören u.a. Aldehyde und Ketone, insbesondere Aceton, welches zur Herstellung des Acetoncyanhydrins eingesetzt wurde. Dies wird beispielsweise in US 4018829-A beschrieben. Die Reinheit der zugegebenen Aldehyde und/oder Ketone ist im Allgemeinen nicht besonders kritisch. Dementsprechend können diese Stoffe Verunreinigungen, insbesondere Alkohole, beispielsweise Methanol, Wasser und/oder 2-Hydroxyisobuttersäuremethylester (HIBSM) enthalten. Die Menge an Carbonylverbindungen, insbesondere Aceton und / oder Acetaldehyd kann in der Reaktionsmischung in weiten Bereichen eingesetzt werden. Vorzugsweise wird die Carbonylverbindung in einer Menge im Bereich von 0,1-6 Mol, vorzugsweise 0,1-2 Mol pro Mol Cyanhydrin eingesetzt.

Die Temperatur, bei der Hydrolysereaktion erfolgt, kann im Allgemeinen im Bereich von 10-150°C, vorzugsweise im Bereich von 20-100°C und besonders bevorzugt im Bereich von 30-80°C liegen.

Die Reaktion kann beispielsweise in einem Festbettreaktor oder in einem Suspensionsreaktor durchgeführt werden.

Die so erhaltene Reaktionsmischung umfasst im Allgemeinen neben dem gewünschten 2-Hydroxyisobuttersäureamid weitere Bestandteile, insbesondere nicht umgesetztes Acetoncyanhydrin sowie gegebenenfalls eingesetztes Aceton und / oder Acetaldehyd. Dementsprechend kann die Reaktionsmischung aufgereinigt werden, wobei nicht umgesetztes Acetoncyanhydrin in Aceton und Blausäure gespalten werden kann, um diese wieder zur Herstellung des Cyanhydrins einzusetzen. Gleiches gilt für das abgetrennte Aceton und / oder Acetaldehyd.

Des Weiteren kann die aufgereinigte, Hydroxysäureamid umfassende Reaktionsmischung durch Ionenaustauschsäulen von weiteren Bestandteilen gereinigt werden.

Hierzu können insbesondere Kationenaustauscher und Anionenaustauscher eingesetzt werden. Hierfür geeignete Ionenaustauscher sind an sich bekannt. Beispielsweise können geeignete Kationenaustauscher durch Sulfonierung von Styrol-Divinylbenzolcopolymeren erhalten werden. Basische Anionenaustauscher umfassen quaternäre Ammoniumgruppen, die kovalent an Styrol-Divinylbenzolcopolymere gebunden sind.

Die Schritte zur Herstellung von α-Hydroxycarbonsäureamiden sind u.a. in EP-A-0686623 detaillierter beschrieben.

Im nächsten Schritt C) kann das so erhaltene 2-Hydroxyisobuttersäureamid zum 2-Hydroxycarbonsäurealkylester umgesetzt werden. Dies kann beispielsweise durch die Verwendung von Alkylformiaten erfolgen. Insbesondere geeignet ist Methylformiat oder eine Mischung von Methanol und Kohlenmonooxid, wobei diese Reaktion beispielhaft in EP-A-0407811 beschrieben ist.

Bevorzugt erfolgt die Umsetzung des 2-Hydroxyisobuttersäureamids durch Alkoholyse mit einem Alkohol, der vorzugsweise 1-10 Kohlenstoffatome, besonders bevorzugt 1 bis 5 Kohlenstoffatome umfasst. Bevorzugte Alkohole sind u.a. Methanol, Ethanol, Propanol, Butanol, insbesondere n-Butanol und 2-Methyl-1-propanol, Pentanol, Hexanol, Heptanol, 2-Ethylhexanol, Octanol, Nonanol und Decanol. Besonders bevorzugt wird als Alkohol Methanol und / oder Ethanol eingesetzt, wobei Methanol ganz besonders bevorzugt ist. Die Umsetzung von Carbonsäureamiden mit Alkoholen, um Carbonsäureester zu erhalten, ist allgemein bekannt.

Diese Reaktion kann beispielsweise durch basische Katalysatoren beschleunigt werden. Diese umfassen homogene Katalysatoren sowie heterogene Katalysatoren.

Zu den homogenen Katalysatoren gehören Alkalimetallalkoholate und organometallische Verbindungen von Titan, Zinn und Aluminium. Vorzugsweise wird ein Titanalkoholat oder Zinnalkoholat, wie beispielsweise Titantetraisopropyloxid oder Zinntetrabutyloxid eingesetzt. Zu den heterogenen Katalysatoren gehören u.a. Magnesiumoxid, Calciumoxid sowie basische Ionenaustauscher, wie sie zuvor beschrieben wurden.

Das Molverhältnis von 2-Hydroxyisobuttersäureamid zu Alkohol, beispielsweise Methanol, ist an sich nicht kritisch, wobei dieses vorzugsweise im Bereich von 2:1-1:20 liegt.

Die Reaktionstemperatur kann ebenfalls in weiten Bereichen liegen, wobei die Reaktionsgeschwindigkeit mit zunehmender Temperatur im Allgemeinen zunimmt. Die obere Temperaturgrenze ergibt sich im Allgemeinen aus dem Siedepunkt des eingesetzten Alkohols. Vorzugsweise liegt die Reaktionstemperatur im Bereich von 40-300°C, besonders bevorzugt 160-240°C. Die Reaktion kann, je nach Reaktionstemperatur, bei Unter- oder Überdruck durchgeführt werden. Vorzugsweise wird diese Reaktion in einem Druckbereich von 0,5-35 bar, besonders bevorzugt 5 bis 30 bar durchgeführt.

Üblicherweise wird der entstehende Ammoniak aus dem Reaktionssystem abgeleitet, wobei die Umsetzung vielfach beim Siedepunkt durchgeführt wird.

Der bei der Alkoholyse freigesetzte Ammoniak kann dem Gesamtprozess auf leichte Weise zurückgeführt werden. Beispielsweise kann Ammoniak mit Methanol zu Blausäure umgesetzt werden. Dies ist beispielsweise in EP-A-0941984 dargelegt. Des Weiteren kann die Blausäure aus Ammoniak und Methan gemäß dem BMA- oder Andrussow-Verfahren erhalten werden, wobei diese Verfahren in Ullmann's Encyclopedia of Industrial Chemistry 5. Auflage auf CD-ROM, Stichwort "Inorganic Cyano Compounds" beschrieben sind.

In einem nächsten Schritt D) wird der 2-Hydroxyisobuttersäureesters mit Methacrylsäure (2-Methylpropensäure) umgesetzt, wobei Alkylmethacrylat sowie 2- Hydroxyisobuttersäure erhalten werden.

Gemäß dem weiteren Aspekt der vorliegenden Erfindung können 2-Hydroxyisobuttersäureester mit Methacrylsäure umgesetzt werden. Die hierfür eingesetzten 2-Hydroxyisobuttersäureester sind an sich bekannt, wobei der Alkoholrest des Esters vorzugsweise 1 bis 20 Kohlenstoffatome, insbesondere 1 bis 10 Kohlenstoffatome und besonders bevorzugt 1 bis 5 Kohlenstoffatome umfasst. Bevorzugte Alkoholreste leiten sich insbesondere von Methanol, Ethanol, Propanol, Butanol, insbesondere n-Butanol und 2-Methyl-1-propanol, Pentanol, Hexanol und 2-Ethylhexanol ab, wobei Methanol und Ethanol besonders bevorzugt sind.

Bevorzugt eingesetzte 2-Hydroxyisobuttersäureester sind α-Hydroxyisobuttersäuremethylester und α-Hydroxyisobuttersäureethylester.

Die Reaktionsmischung kann neben den Reaktanden weitere Bestandteile, wie beispielsweise Lösungsmittel, Katalysatoren, Polymerisationsinhibitoren und Wasser umfassen.

Die Umsetzung des 2-Hydroxyisobuttersäureesters mit Methacrylsäure kann durch mindestens eine Säure oder mindestens eine Base katalysiert werden. Hierbei können sowohl homogene als auch heterogene Katalysatoren verwendet werden. Bevorzugte saure Katalysatoren wurden zuvor dargelegt, wobei insbesondere Kationenaustauscherharzen, wie sulfonsäuregruppenhaltige Styrol-Divinylbenzolpolymere besonders geeignet sind.

Zu den besonders geeigneten Kationenaustauscherharzen gehören insbesondere sulfonsäuregruppenhaltige Styrol-Divinylbenzolpolymere. Besonders geeignete Kationenaustauscherharze können kommerziell von Rohm&Haas unter der Handelsbezeichnung Amberlyst® und von Lanxess unter der Handelsbezeichnung Lewatit® erhalten werden.

Die Konzentration an Katalysator liegt vorzugsweise im Bereich von 1 bis 30 Gew.-%, besonders bevorzugt 5 bis 15 Gew.-%, bezogen auf die Summe des eingesetzten 2-Hydroxyisobuttersäureesters und der eingesetzten Methacrylsäure.

Zu den bevorzugt einsetzbaren Polymerisationsinhibitoren gehören unter anderem Phenothiazin, Tertiärbutylcatechol, Hydrochinonmonomethylether, Hydrochinon, 4-Hydroxy-2,2,6,6-tetramethylpiperidinooxyl (TEMPOL) oder deren Gemische; wobei die Wirksamkeit dieser Inhibitoren durch Einsatz von Sauerstoff teilweise verbessert werden kann. Die Polymerisationsinhibitoren können in einer Konzentration im Bereich von 0,001 bis 2,0 Gew.-%, besonders bevorzugt im Bereich von 0,01 bis 0,2 Gew.-%, bezogen auf die Summe des eingesetzten 2-Hydroxyisobuttersäureesters und der eingesetzten Methacrylsäure, eingesetzt werden.

Die Reaktion wird bevorzugt bei Temperaturen im Bereich von 50°C bis 200°C, besonders bevorzugt 70°C bis 130°C, insbesondere 80°C bis 120°C und ganz besonders bevorzugt 90°C bis 110°C durchgeführt.

Die Reaktion kann bei Unter- oder Überdruck durchgeführt werden, je nach Reaktionstemperatur. Vorzugsweise wird diese Reaktion bei einem Druck im Bereich von 0,02-5 bar, insbesondere 0,2 bis 3 bar und besonders bevorzugt 0,3 bis 0,5 bar durchgeführt.

Das molare Verhältnis von Methacrylsäure zum 2-Hydroxyisobuttersäureester liegt vorzugsweise im Bereich von 4:1-1:4, insbesondere 3:1 bis 1:3 und besonders bevorzugt im Bereich von 2:1-1:2.

Bevorzugt beträgt die Selektivität mindestens 90 %, besonders bevorzugt 98 %. Die Selektivität ist definiert als das Verhältnis der Summe aus gebildeten Stoffmengen an Alkylmethacrylaten und 2-Hydroxyisobuttersäure, bezogen auf die Summe der umgesetzten Stoffmengen an 2-Hydroxyisobuttersäureester und Methacrylsäure.

Gemäß einem besonderen Aspekt der vorliegenden Erfindung kann die Umesterung in Gegenwart von Wasser erfolgen. Vorzugsweise liegt der Wassergehalt im Bereich von 0,1-50 Gew.-%, besonders bevorzugt 0,5-20 Gew.-%, und ganz besonders bevorzugt 1-10 Gew.-%, bezogen auf das Gewicht des eingesetzten 2-Hydroxyisobuttersäureesters.

Durch den Zusatz von geringen Mengen Wasser kann überraschend die Selektivität der Umsetzung erhöht werden. Trotz Wasserzugabe kann dabei die Bildung von Methanol überraschend gering gehalten werden. Bei einer Wasserkonzentration von 10 bis 15 Gew.-%, bezogen auf das Gewicht des eingesetzten 2-Hydroxyisobuttersäureesters, bilden sich vorzugsweise weniger als 5 Gew.-% Methanol bei einer Reaktionstemperatur von 120°C und einer Reaktionsdauer bzw. Verweilzeit von 5 bis 180 min.

Die Umesterung kann chargenweise oder kontinuierlich durchgeführt werden, wobei kontinuierliche Verfahren bevorzugt sind.

Die Reaktionsdauer der Umesterung hängt von den eingesetzten Molmassen sowie der Reaktionstemperatur ab, wobei dieser Parameter in weiten Bereichen liegen kann. Bevorzugt liegt die Reaktionszeit der Umesterung des 2-Hydroxyisobuttersäureesters mit Methacrylsäure im Bereich von 30 Sekunden bis 15 Stunden, besonders bevorzugt 5 Minuten bis 5 Stunden und ganz besonders bevorzugt 15 Minuten bis 3 Stunden.

Bei kontinuierlichen Verfahren beträgt die Verweilzeit vorzugsweise 30 Sekunden bis 15 Stunden, besonders bevorzugt 5 Minuten bis 5 Stunden und ganz besonders bevorzugt 15 Minuten bis 3 Stunden.

Bei der Herstellung von Methylmethacrylat aus α-Hydroxyisobuttersäuremethylester beträgt die Temperatur vorzugsweise 60 bis 130 °C, besonders bevorzugt 80 bis 120°C und ganz besonders bevorzugt 90 bis 110 °C. Der Druck liegt vorzugsweise im Bereich von 50 bis 1000 mbar, besonders bevorzugt 300 bis 800 mbar. Das molare Verhältnis von Methacrylsäure zu α-Hydroxyisobuttersäuremethylester liegt vorzugsweise im Bereich von 2:1-1:2, insbesondere 1,5:1-1:1,5.

Gemäß einer besonders bevorzugten Ausführungsform kann die Umesterung in einer Destille erfolgen. Hierbei kann der Katalysator in jedem Bereich der Destille beigefügt werden. Beispielsweise kann der Katalysator in den Bereich des Sumpfes oder im Bereich der Kolonne bereitgestellt werden. Hierbei sollten die Edukte jedoch in Kontakt mit dem Katalysator gebracht werden. Des Weiteren kann Katalysator in einem separaten Bereich der Destille bereitgestellt werden, wobei dieser Bereich mit den weiteren Bereichen der Destille, beispielsweise des Sumpfes und/oder der Kolonne verbunden sind. Diese separate Anordnung des Katalysatorbereichs ist bevorzugt.

Durch diese bevorzugte Ausgestaltung gelingt es überraschend die Selektivität der Umsetzung zu erhöhen. In diesem Zusammenhang ist festzuhalten, dass der Druck der Umsetzung unabhängig vom Druck innerhalb der Destillationskolonnen eingestellt werden kann. Hierdurch kann die Siedetemperatur niedrig gehalten werden, ohne dass die Reaktionszeit bzw. die Verweilzeit entsprechend ansteigt. Darüber hinaus kann die Temperatur der Umsetzung über einen weiten Bereich variiert werden. Hierdurch kann die Reaktionszeit verkürzt werden. Darüber hinaus kann das Volumen an Katalysator beliebig gewählt werden, ohne dass auf die Geometrie der Kolonne Rücksicht genommen werden müsste. Weiterhin kann beispielsweise ein weiterer Reaktand hinzugefügt werden. All diese Maßnahmen können zur Steigerung der Selektivität und der Produktivität beitragen, wobei überraschende Synergieeffekte erzielt werden.

Hierbei wird 2-Hydroxyisobuttersäuremethylester der Destille zugeführt. Des Weiteren wird Methacrylsäure in die Destille eingeleitet. Die Destillationsbedingungen werden bevorzugt so ausgeführt, dass genau ein Produkt durch Destillation aus der Destille abgeleitet wird, wobei das zweite Produkt im Sumpf verbleibt und aus diesem kontinuierlich entfernt wird. Bei Verwendung von Alkoholen mit einer geringen Kohlenstoffänzahl, insbesondere Ethanol oder Methanol, wird vorzugsweise das Alkylmethacrylat durch Destillation der Reaktionsmischung entzogen. Die Edukte werden zyklisch durch den Katalysatorbereich geleitet. Hierdurch entsteht kontinuierlich Alkylmethacrylat sowie 2-Hydroxyisobuttersäure.

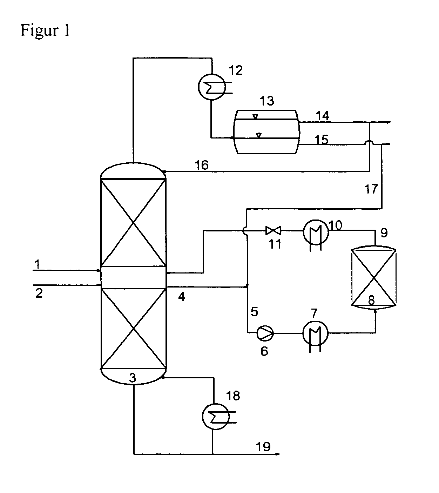

Eine bevorzugte Ausführungsform einer Destille ist in Fig. 1 schematisch dargelegt. Die Edukte können über eine gemeinsame Leitung (1) oder getrennt über zwei Leitungen (1) und (2) in die Destillationskolonne (3) eingeleitet werden. Bevorzugt erfolgt die Zugabe der Edukte über getrennte Leitungen. Die Edukte können dabei auf derselben Stufe oder in beliebiger Position der Kolonne zugeführt werden.

Die Temperatur der Reaktanden kann dabei über einen Wärmetauscher in der Zuführung eingestellt werden, wobei die hierfür notwendigen Aggregate nicht in Figur 1 dargestellt sind. In einer bevorzugten Variante werden die Edukte separat in die Kolonne dosiert, wobei die Zudosierung der leichtersiedenden Komponente unterhalb der Position für die Zuführung der schwersiedenden Verbindung erfolgt. Bevorzugt wird in diesem Fall die leichtersiedende Komponente dampfförmig zugegeben.

Für die vorliegende Erfindung kann jede mehrstufige Destillationskolonne (3) verwendet werden, die zwei oder mehr Trennstufen besitzt. Als Anzahl der Trennstufen wird in der vorliegenden Erfindung die Anzahl der Böden bei einer Bodenkolonne oder die Anzahl der theoretischen Trennstufen im Fall einer Packungskolonne oder eine Kolonne mit Füllkörpern bezeichnet.

Beispiele für eine mehrstufige Destillationskolonne mit Böden beinhalten solche wie Glockenböden, Siebböden, Tunnelböden, Ventilböden, Schlitzböden, Sieb-Schlitzböden, Sieb-Glockenböden, Düsenböden, Zentrifugalböden, für eine mehrstufige Destillationskolonne mit Füllkörpern solche wie Raschig-Ringe, Lessing-Ringe, Pall-Ringe, Berl-Sättel, Intalox Sättel und für eine mehrstufige Destillationskolonne mit Packungen wie solche vom Typ Mellapak (Sulzer), Rombopak (Kühni), Montz-Pak (Montz) und Packungen mit Katalysatortaschen, beispielsweise Kata-Pak.

Eine Destillationskolonne mit Kombinationen aus Bereichen von Böden, aus Bereichen von Füllkörpern oder aus Bereichen von Packungen kann ebenso verwendet werden.

Die Kolonne (3) kann mit Einbauten ausgestattet sein. Die Kolonne weist vorzugsweise einen Kondensator (12) zur Kondensation des Dampfes und einen Sumpf-Verdampfer (18) auf.

Vorzugsweise weist die Destillationsapparatur mindestens einen Bereich, nachfolgend Reaktor genannt, auf, in dem mindestens ein Katalysator vorgesehen ist. Dieser Reaktor kann innerhalb der Destillationskolonne liegen. Vorzugsweise wird dieser Reaktor jedoch außerhalb der Kolonne (3) in einem separaten Bereich angeordnet, wobei eine dieser bevorzugten Ausführungsformen in Figur 1 näher erläutert wird.

Um die Umesterungsreaktion in einem separaten Reaktor (8) durchzuführen, kann innerhalb der Kolonne ein Teil der abwärts strömenden flüssigen Phase über einen Sammler aufgefangen und als Teilstrom (4) aus der Kolonne geleitet. Die Position des Sammlers wird durch das Konzentrationsprofil in der Kolonne der einzelnen Komponenten bestimmt. Das Konzentrationsprofil kann hierbei über die Temperatur und/oder den Rücklauf geregelt werden. Der Sammler wird bevorzugt so positioniert, dass der aus der Kolonne herausgeführte Strom beide Reaktanden, besonders bevorzugt die Reaktanden in ausreichend hoher Konzentration und ganz besonders bevorzugt im molaren Verhältnis Säure : Ester = 1,5:1 bis 1:1,5 enthält. Des Weiteren können mehrere Sammler an verschiedenen Stellen der Destillationskolonne vorgesehen sein, wobei durch die Menge an entnommenen Reaktanden die molaren Verhältnisse eingestellt werden können.

Dem aus der Kolonne abgeführten Strom kann zudem noch ein weiterer Reaktand, beispielsweise Wasser zudosiert werden, um das Produktverhältnis Säure/Ester in der Kreuzumesterungsreaktion einzustellen oder die Selektivität zu erhöhen. Das Wasser kann über eine Leitung von außen zugeführt (in Figur 1 nicht dargestellt) oder aus einem Phasentrenner (13) entnommen werden. Der Druck des mit Wasser angereicherten Stroms (5) kann anschließend über ein Mittel zur Druckerhöhung (6), beispielsweise eine Pumpe, erhöht werden.

Durch eine Erhöhung des Drucks kann eine Bildung von Dampf in dem Reaktor, beispielsweise einem Festbettreaktor vermindert bzw. verhindert werden. Hierdurch kann eine gleichmäßige Durchströmung des Reaktors und Benetzung der Katalysatorpartikel erzielt werden. Der Strom kann durch einen Wärmetauscher (7) geführt und die Reaktionstemperatur eingestellt werden. Der Strom kann dabei je nach Bedarf erwärmt oder gekühlt werden. Über die Reaktionstemperatur kann zudem das Produktverhältnis Ester zu Säure eingestellt werden.

Im Festbettreaktor (8) findet am Katalysator die Umesterungsreaktion statt. Der Reaktor kann dabei abwärts oder aufwärts durchströmt werden. Der die zu einem gewissen Anteil die Produkte und die nicht umgesetzten Edukte enthaltende Reaktorabstrom (9), wobei der Anteil der Komponenten im Reaktorabstrom von der Verweilzeit, der Katalysatormasse, der Reaktionstemperatur und dem Eduktverhältnis sowie der zugesetzten Wassermenge abhängt, wird zunächst durch einen Wärmetauscher (10) geleitet und auf eine Temperatur eingestellt, die beim Einleiten in die Destillationskolonne vorteilhaft ist. Bevorzugt wird die Temperatur eingestellt, die der Temperatur in der Destillationskolonne an der Stelle des Einleitens des Stroms entspricht.

Die Position, wo der den Reaktor verlassende Strom in die Kolonne zurückgeführt wird, kann dabei ober- oder unterhalb der Position für die Entnahme der Reaktorzuführung liegen, bevorzugt wird jedoch oberhalb. Vor dem Rückführen in die Kolonne kann der Strom über ein Ventil (11) entspannt werden, wobei vorzugsweise das gleiche Druckniveau wie in der Kolonne eingestellt wird. Bevorzugt weist die Destillationskolonne hierbei einen niedrigeren Druck auf. Diese Ausgestaltung bietet den Vorteil, dass die Siedepunkte der zu trennenden Komponenten herabgesetzt werden, wodurch die Destillation auf niedrigerem Temperaturniveau, dadurch energiesparender und thermisch schonender durchgeführt werden kann.

In der Destillationskolonne (3) erfolgt dann die Auftrennung des Produktgemisches. Der Niedersieder, bevorzugt der in der Umesterung gebildete Ester, wird über Kopf abgetrennt. Bevorzugt wird die Destillationskolonne so gefahren, dass das vor dem Festbettreaktor zugegebene Wasser ebenfalls als Kopfprodukt abgetrennt wird. Der am Kopf abgezogene, dampfförmige Strom wird in einem Kondensator (12) kondensiert und anschließend in einem Dekanter (13) in die wässrige und Produktesterhaltige Phase aufgetrennt. Die wässrige Phase kann über die Leitung (15) zur Aufarbeitung ausgeschleust oder vollständig oder teilweise über Leitung (17) wieder in die Reaktion zurückgeführt werden. Der Strom aus der esterhaltigen Phase kann über Leitung (14) zum Teil als Rücklauf (16) auf die Kolonne gefahren werden oder zum Teil aus der Destille ausgeschleust werden. Der Schwersieder, bevorzugt die in der Kreuzumesterung gebildete Säure, wird als Sumpfstrom aus der Kolonne (19) ausgeschleust.

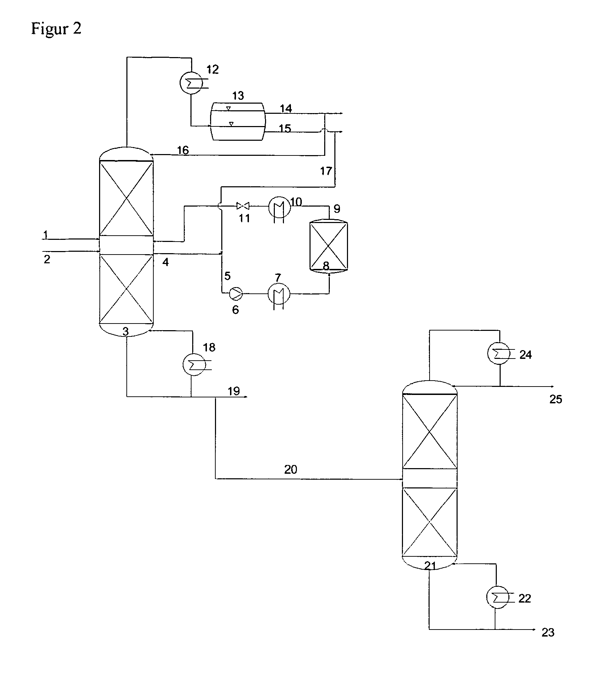

Eine weitere Ausführungsform einer besonderen Reaktivdestille ist in Figur 2 dargestellt, wobei diese der zuvor dargelegten Ausführungsform im Wesentlichen ähnelt, so dass nachstehend lediglich auf die Unterschiede eingegangen wird, wobei gleiche Bezugszeichen für gleiche Teile verwendet werden und die obige Beschreibung entsprechend gilt.

Die in Figur 2 dargestellte Reaktivdestille weist eine zweite Destillationskolonne (21) auf, die über Leitung (20) mit der zuvor beschriebenen Leitung (19) oder mit dem Sumpf der Destillationskolonne (3) verbunden ist. Je nach Verweilzeit in dem Sumpf der Destillationskolonne (3) kann die entnommene Zusammensetzung mehr oder minder große Mengen an 2-Hydroxyisobuttersäure enthalten. Im Sumpf der mit einem Sumpf-Verdampfer (22) ausgestatteten Destillationskolonne (21) kann die 2-Hydroxyisobuttersäure in Tetramethylglycolid überführt und über Leitung 23 der Anlage entnommen werden. In Destillationskolonne (21) kann aus der Zusammensetzung Wasser abgezogen, mit einem Kondensator (24) kondensiert werden und der Destille entnommen werden.

Falls die im Sumpf der Destillationskolonne (3) enthaltene 2-Hydroxyisobuttersäure in hoher Menge in Tetramethylglycolid überführt wurde, ermöglicht diese Anlage ebenfalls eine Aufreinigung des erhaltenen Tetramethylglycolids. Hierbei kann Tetramethylglycolid über den Kopf entnommen werden. Der aus dem Sumpf entnommene Strom umfasst einen in Bezug auf den Zugabestrom hohen Anteil an 2-Hydroxyisobuttersäure.

Die aus der Reaktion erhaltene 2-Hydroxyisobuttersäure, kann auf bekannte Weise in einem weiteren Schritt E) dehydratisiert werden. Im Allgemeinen wird die α-Hydroxyisobuttersäure in Gegenwart mindestens eines Metallsalzes, beispielsweise von Alkali- und/oder Erdalkalimetallsalzen, auf Temperaturen im Bereich von 160-300°C, besonders bevorzugt im Bereich von 200 bis 240 °C erhitzt, wobei im Allgemeinen die Methacrylsäure sowie Wasser erhalten werden. Zu den geeigneten Metallsalzen gehören u.a. Natriumhydroxid, Kaliumhydroxid, Calciumhydroxid, Bariumhydroxid, Magnesiumhydroxid, Natriumsulfit, Natriumcarbonat, Kaliumcarbonat, Strontiumcarbonat, Magnesiumcarbonat, Natriumbicarbonat, Natriumacetat, Kaliumacetat und Natriumdihydrogenphosphat.

Die Dehydratisierung der α-Hydroxycarbonsäure kann vorzugsweise bei einem Druck im Bereich von 0,05 bar bis 2,5 bar, besonders bevorzugt im Bereich von 0,1 bar bis 1 bar durchgeführt werden.

Die Dehydratisierung von α-Hydroxycarbonsäuren ist beispielsweise in DE-A-176 82 53 beschrieben.

Die so erhaltene Methacrylsäure kann wiederum zur Herstellung von Alkylmethacrylaten eingesetzt werden. Des Weiteren ist Methacrylsäure ein Handelsprodukt. Überraschend kann dementsprechend das Verfahren zur Herstellung von Alkylmethacrylaten ebenfalls dazu dienen, Methacrylsäure herzustellen, wobei das Produktverhältnis von Alkyl(meth)acrylaten zu Methacrylsäure durch die Konzentration an Wasser bei der Umesterung des α-Hydroxycarbonsäurealkylesters und/oder durch die Reaktionstemperatur leicht reguliert werden kann.

Nachfolgend soll die vorliegende Erfindung anhand von Beispielen näher erläutert werden.

### Beispiel 1

In eine Destille mit Kolonne und Kondensator wurden 1,2 kg (11,54 mol) reine 2-Hydroxyisobuttersäure (HIBS) gegeben. Der Druck wurde auf 400 mbar und die Reaktionstemperatur wurde auf ca. 180°C eingestellt, wobei die Sumpftemperaturen in Tabelle 1 angegeben sind. Kontinuierlich wurde durch Destillation Wasser aus der Reaktionsmischung abgetrennt. Die Temperatur an Kopf der Destille betrug ca. 76°C. Nach den in Tabelle 1 dargelegten Zeitabständen wurden aus dem Sumpf Proben entnommen und mittels Gaschromatographie analysiert.

**Tabelle 1**

| Zeit | Temperatur des Sumpfes | Tetramethylglycolid | HIBS |
|---|---|---|---|
| [min] | [°C] | [Gew.-%] | [Gew.-%] |
| 79 | 178 | 40,19 | 58,07 |
| 139 | 180 | 56,04 | 42,95 |
| 199 | 184 | 74,48 | 23,72 |
| 259 | 185 | 80,84 | 18,25 |
| 334 | 188 | 95,20 | 2,63 |
| 414 | 187 | 95,45 | 2,70 |
| 504 | 188 | 97,60 | 0,23 |
| 594 | 182 | 98,19 | 0,24 |
| 604 | 182 | 98,27 | 0,23 |

Überraschend konnte gezeigt werden, dass bei Verwendung von 2-Hydroxyisobuttersäure als Edukt im Wesentlichen reines Tetramethylglycolid als cyclischer Ester gebildet wird.

## Patentansprüche

1. Verfahren zur Herstellung von Tetramethylglycolid, **dadurch gekennzeichnet, dass** eine Zusammensetzung, die mindestens 50 Gew.-% 2-Hydroxyisobuttersäure und/oder Tetramethylglycolid umfasst, auf eine Temperatur von mindestens 100°C erhitzt wird und die Umsetzung bei einem Druck im Bereich von 0,1 bis 0,4 bar durchgeführt wird.

2. Verfahren gemäß Anspruch 1, **dadurch gekennzeichnet, dass** die Zusammensetzung mindestens 70 Gew.-% 2-Hydroxyisobuttersäure und/oder Tetramethylglycolid umfasst.

3. Verfahren gemäß Anspruch 1 oder 2, **dadurch gekennzeichnet, dass** die Umsetzung in einer Destille durchgeführt wird.

4. Verfahren gemäß Anspruch 3, **dadurch gekennzeichnet, dass** entstehendes Wasser aus der Reaktionsmischung durch Destillation entfernt wird.

5. Verfahren gemäß mindestens einem der vorhergehenden Ansprüche, **dadurch gekennzeichnet, dass** die Umsetzung bei einer Temperatur von mindestens 150°C erfolgt.

6. Verfahren gemäß mindestens einem der vorhergehenden Ansprüche, **dadurch gekennzeichnet, dass** die Umsetzung kontinuierlich durchgeführt wird.

7. Verfahren gemäß mindestens einem der vorhergehenden Ansprüche, **dadurch gekennzeichnet, dass** die Umsetzung autokatalytisch erfolgt.

8. Verfahren gemäß mindestens einem der vorhergehenden Ansprüche, **dadurch gekennzeichnet, dass** das erhaltene Tetramethylglycolid durch einen Destillationsschritt aufgereinigt wird.

9. Verfahren gemäß mindestens einem der vorhergehenden Ansprüche, **dadurch gekennzeichnet, dass** das erhaltene Tetramethylglycolid durch Extraktion aufgereinigt wird.

10. Verfahren gemäß mindestens einem der vorhergehenden Ansprüche, **dadurch gekennzeichnet, dass** das Verfahren in Kombination mit einem Verfahren zur Herstellung von Alkylmethacrylaten durchgeführt wird.

11. Verfahren gemäß Anspruch 10, **dadurch gekennzeichnet, dass** das Verfahren die folgenden Schritte umfasst
A) Bildung von Acetoncyanhydrin durch Umsetzung von Aceton mit Blausäure;
B) Hydrolyse des Acetoncyanhydrins unter Bildung von 2-Hydroxyisobuttersäureamid;
C) Alkoholyse des 2-Hydroxyisobuttersäureamids, wobei ein 2-Hydroxyisobuttersäureester erhalten wird;
D) Umesterung des 2-Hydroxyisobuttersäureesters bzw. der 2-Hydroxyisobuttersäureester mit Methacrylsäure, wobei mindestens ein Alkylmethacrylat und 2-Hydroxyisobuttersäure gebildet werden;
E) Dehydratisierung der 2-Hydroxyisobuttersäure, wobei Methacrylsäure gebildet wird.

12. Verfahren gemäß Anspruch 11, **dadurch gekennzeichnet, dass** die Umesterung des α-Hydroxycarbonsäurealkylesters mit Methacrylsäure durch eine Säure katalysiert wird.

13. Verfahren gemäß Anspruch 12, **dadurch gekennzeichnet, dass** die Säure ein Ionenaustauscher ist.

14. Verfahren gemäß mindestens einem der Ansprüche 11 bis 13, **dadurch gekennzeichnet, dass** die Umesterung in einer Destille durchgeführt wird.

15. Verfahren gemäß mindestens einem der vorhergehenden Ansprüche 11 bis 14, **dadurch gekennzeichnet, dass** die Umesterung des α-Hydroxyisobuttersäureesters mit Methacrylsäure bei einem Druck im Bereich von 100 mbar bis 3 bar durchgeführt wird.

16. Verfahren gemäß mindestens einem der vorhergehenden Ansprüche 11 bis 15, **dadurch gekennzeichnet, dass** die Umesterung des α-Hydroxyisobuttersäureesters mit Methacrylsäure bei einer Temperatur im Bereich von 70 bis 130°C durchgeführt wird.

17. Verfahren gemäß mindestens einem der vorhergehenden Ansprüche 11 bis 16, **dadurch gekennzeichnet, dass** die Umesterung des 2-Hydroxyisobuttersäureesters mit Methacrylsäure in Gegenwart von Wasser durchgeführt wird.

18. Verfahren gemäß mindestens einem der vorhergehenden Ansprüche 10 bis 17, **dadurch gekennzeichnet, dass** Methylmethacrylat hergestellt wird.

## Claims

1. Process for preparing tetramethylglycolide, **characterized in that** a composition which comprises at least 50% by weight of 2-hydroxyisobutyric acid and/or tetramethylglycolide is heated to a temperature of at least 100°C and the reaction is performed at a pressure of 0.1 to 0.4 bar.

2. Process according to Claim 1, **characterized in that** the composition comprises at least 70% by weight of 2-hydroxyisobutyric acid and/or tetramethylglycolide.

3. Process according to Claim 1 or 2, **characterized in that** the reaction is performed in a still.

4. Process according to Claim 3, **characterized in that** water formed is removed from the reaction mixture by distillation.

5. Process according to at least one of the preceding claims, **characterized in that** the reaction is effected at a temperature of at least 150°C.

6. Process according to at least one of the preceding claims, **characterized in that** the reaction is performed continuously.

7. Process according to at least one of the preceding claims, **characterized in that** the reaction is effected autocatalytically.

8. Process according to at least one of the preceding claims, **characterized in that** the resulting tetramethylglycolide is purified by a distillation step.

9. Process according to at least one of the preceding claims, **characterized in that** the resulting tetramethylglycolide is purified by extraction.

10. Process according to at least one of the preceding claims, **characterized in that** the process is performed in combination with a process for preparing alkyl methacrylates.

11. Process according to Claim 10, **characterized in that** the process comprises the following steps:
A) formation of acetone cyanohydrin by reacting acetone with hydrocyanic acid;
B) hydrolysis of the acetone cyanohydrin to form 2-hydroxyisobutyramide;
C) alcoholysis of the 2-hydroxyisobutyramide to obtain a 2-hydroxyisobutyric ester;
D) transesterification of the 2-hydroxyisobutyric ester or of the 2-hydroxyisobutyric esters with methacrylic acid to form at least one alkyl methacrylate and 2-hydroxyisobutyric acid;
E) dehydration of the 2-hydroxyisobutyric acid to form methacrylic acid.

12. Process according to Claim 11, **characterized in that** the transesterification of the alkyl α-hydroxycarboxylate with methacrylic acid is catalysed by an acid.

13. Process according to Claim 12, **characterized in that** the acid is an ion exchanger.

14. Process according to at least one of Claims 11 to 13, **characterized in that** the transesterification is performed in a still.

15. Process according to at least one of the preceding Claims 11 to 14, **characterized in that** the transesterification of the α-hydroxyisobutyric ester with methacrylic acid is performed at a pressure in the range of 100 mbar to 3 bar.

16. Process according to at least one of the preceding Claims 11 to 15, **characterized in that** the transesterification of the α-hydroxyisobutyrate with methacrylic acid is performed at a temperature in the range of 70 to 130°C.

17. Process according to at least one of the preceding Claims 11 to 16, **characterized in that** the transesterification of the 2-hydroxyisobutyric ester with methacrylic acid is performed in the presence of water.

18. Process according to at least one of the preceding Claims 10 to 17, **characterized in that** methyl methacrylate is prepared.

## Revendications

1. Procédé pour la préparation de tétraméthylglycolide, **caractérisé en ce qu'**on chauffe une composition qui contient au moins 50% en poids d'acide 2-hydroxyisobutyrique et/ou de tétraméthylglycolide, à une température d'au moins 100°C et la transformation est réalisée à une pression dans la plage de 0,1 à 0,4 bars.

2. Procédé selon la revendication 1, **caractérisé en ce que** la composition contient au moins 70% en poids d'acide 2-hydroxyisobutyrique et/ou de tétraméthylglycolide.

3. Procédé selon la revendication 1 ou 2, **caractérisé en ce que** la transformation est réalisée dans un appareil de distillation.

4. Procédé selon la revendication 3, **caractérisé en ce que** l'eau qui se forme est éliminée par distillation du mélange réactionnel.

5. Procédé selon au moins l'une quelconque des revendications précédentes, **caractérisé en ce que** la transformation est réalisée à une température d'au moins 150°C.

6. Procédé selon au moins l'une quelconque des revendications précédentes, **caractérisé en ce que** la transformation est réalisée en continu.

7. Procédé selon au moins l'une quelconque des revendications précédentes, **caractérisé en ce que** la transformation a lieu par voie autocatalytique.

8. Procédé selon au moins l'une quelconque des revendications précédentes, **caractérisé en ce que** le tétraméthylglycolide obtenu est purifié par une étape de distillation.

9. Procédé selon au moins l'une quelconque des revendications précédentes, **caractérisé en ce que** le tétraméthylglycolide obtenu est purifié par extraction.

10. Procédé selon au moins l'une quelconque des revendications précédentes, **caractérisé en ce que** le procédé est réalisé en combinaison avec un procédé de préparation de méthacrylates d'alkyle.

11. Procédé selon la revendication 10, **caractérisé en ce que** le procédé comprend les étapes suivantes :
A) formation d'acétonecyanhydrine par transformation d'acétone avec de l'acide cyanhydrique ;
B) hydrolyse de l'acétonecyanhydrine avec formation d'amide de l'acide 2-hydroxyisobutyrique ;
C) alcoolyse de l'amide de l'acide 2-hydroxyisobutyrique, un ester de l'acide 2-hydroxyisobutyrique étant obtenu ;
D) transestérification de l'ester de l'acide 2-hydroxyisobutyrique ou, selon le cas, des esters de l'acide 2-hydroxyisobutyrique avec de l'acide méthacrylique, au moins un méthacrylate d'alkyle e t de l'acide 2-hydroxyisobutyrique étant obtenus ;
E) déshydrogénation de l'acide 2-hydroxyisobutyrique, de l'acide méthacrylique étant formé.

12. Procédé selon la revendication 11, **caractérisé en ce que** la transformation de l'ester alkylique de l'acide α-hydroxycarboxylique avec de l'acide méthacrylique est catalysée par un acide.

13. Procédé selon la revendication 12, **caractérisé en ce que** l'acide est un échangeur ionique.

14. Procédé selon au moins l'une quelconque des revendications 11 à 13, **caractérisé en ce que** la transestérification est réalisée dans un appareil de distillation.

15. Procédé selon au moins l'une quelconque des revendications précédentes 11 à 14, **caractérisé en ce que** la transestérification de l'ester de l'acide α-hydroxyisobutyrique avec de l'acide méthacrylique est réalisée à une pression dans la plage de 100 mbars à 3 bars.

16. Procédé selon au moins l'une quelconque des revendications précédentes 11 à 15, **caractérisé en ce que** la transestérification de l'ester de l'acide α-hydroxyisobutyrique avec de l'acide méthacrylique est réalisée à une température dans la plage de 70 à 130°C.

17. Procédé selon au moins l'une quelconque des revendications précédentes 11 à 16, **caractérisé en ce que** la transestérification de l'ester de l'acide 2-hydroxyisobutyrique avec de l'acide méthacrylique est réalisée en présence d'eau.

18. Procédé selon au moins l'une quelconque des revendications précédentes 10 à 17, **caractérisé en ce que** du méthacrylate de méthyle est préparé.
